# EUROPEAN PATENT APPLICATION

(11) **EP 4 733 756 A2**
(43) Date of publication of application: **29.04.2026**
(21) Application number: 26156112.0
(22) Date of filing: 18.06.2021
(51) Int. Cl.: G01N 30/88

(54) **METHODS OF MEASURING CARFILZOMIB**

(30) Priority: 19.06.2020 US 202063041141 P
(62) Divisional of application: 21746594.7
(71) Applicant: Amgen Inc., Thousand Oaks, California 91320-1799 (US)
(72) Inventor: HARPER, Emma, Thousand Oaks, CA 91320-1799 (US); COHEN, Dawn, Elyse, Thousand Oaks, CA 91320-1799 (US)
(74) Representative: Lau, Sarah Jane

(57) **Abstract**

Provided herein are improved methods for measuring the concentration of carfilzomib in a sample. The method comprises the steps of diluting the sample with a diluent comprising water and less than 50% by volume acetonitrile to form an analytical sample; and subjecting the analytical sample to high performance liquid chromatography (HPLC) to determine the carfilzomib concentration in the sample.

## Description

### BACKGROUND

Accurate assessment of the concentration of an active pharmaceutical ingredient (API) in a solution prior to formulating is important to ensure that the correct amount of the API is portioned into the resulting formulation. Carfilzomib is an approved API for treating multiple myeloma, and is marketed under the name KYPROLIS^{®}. Accurate measurement of carfilzomib during formulating is required to ensure the resulting formulation contains the desired amount of carfilzomib. Thus, a need exists for methods that provide an accurate measurement of carfilzomib in a sample.

### SUMMARY

Provided herein are methods of measuring carfilzomib concentration in a sample during formulation of the carfilzomib. The methods for measuring a carfilzomib concentration in a sample comprise diluting the sample with a diluent comprising water and less than 50% by volume acetonitrile to form an analytical sample; and subjecting the analytical sample to high performance liquid chromatography (HPLC) to obtain the carfilzomib concentration of the sample. In various embodiments, the sample is from carfilzomib compounding. In some embodiments, the sample is from a bulk lyophilization solution of carfilzomib. In some embodiments, the sample is a solubilization sample. In some embodiments, the sample is a post-dilution sample. In various cases, the diluent is water and 0% to 45% by volume acetonitrile. In some cases, the diluent is water and 25% to 40% by volume acetonitrile. In some cases the diluent is water and 30% by volume acetonitrile. In some cases the diluent is water and 40% by volume acetonitrile. In various embodiments, the methods provide a relative standard deviation (RSD) value of less than 2% determined from multiple analytical samples of the sample. In some cases, the RSD% is less than 1%. In some cases, the RSD% is less than 0.5%. In various cases, the concentration of carfilzomib in the sample is 3 mg/mL to 8 mg/mL. In some cases, the concentration of carfilzomib in the sample is 5 mg/mL to 6 mg/mL. In various embodiments, the analytical sample is at a temperature of 2 to 8 C prior to HPLC analysis. In various embodiments, the analytical sample is at room temperature prior to HPLC analysis. In various embodiments, the methods disclosed herein further comprise preparing a lyophilizate from the bulk lyophilization solution. In some cases, the lyophilizate contains 10 mg carfilzomib. In some cases, the lyophilizate contains 30 mg carfilzomib. In some cases, the lyophilizate contains 60 mg carfilzomib.

### BRIEF DESCRIPTION OF THE FIGURES

Figure 1 shows carfilzomib concentration results for samples analyzed using a range of diluents containing varying percentages of acetonitrile by volume in water.
Figure 2 shows concentration of analytical sample solutions that were prepared with 50% v/v ACN diluent, from samples having a concentration at about 7.8 mg/mL, when the analytical sample is at 5°C prior to HPLC analysis, analyzed on Day 1 and Day 2.
Figure 3 shows the concentration of sample solutions ranging from 3.7 mg/mL to 7.8 mg/mL corresponding to a range of 60% to 130% of the method nominal concentration, where the analytical samples were prepared with 30% and 40% v/v ACN diluents and the analytical sample is 5°C prior to HPLC analysis.

### DETAILED DESCRIPTION

Carfilzomib (KYPROLIS^{®}) is approved for treating multiple myeloma and is prepared as a lyophilizate for reconstitution and injection in single dose vials, including vials containing 10 mg, 30 mg, or 60 mg carfilzomib.

Determination of the concentration of carfilzomib prior to formulating it into a lyophilizate is important so that the lyophilizate contains the appropriate amount of carfilzomib. Disclosed herein are improved methods for measuring the concentration of carfilzomib in a sample that provide a more accurate assessment of the carfilzomib in the sample. In some cases, the carfilzomib concentration of the sample is 3 mg/mL to 8 mg/mL. In some cases, the carfilzomib concentration is 5 mg/mL to 6 mg/mL.

The in-process control (IPC) method for bulk (lyophilization) solution of Carfilzomib for Injection is a high-performance liquid chromatography (HPLC) assay used to determine the concentration of carfilzomib in IPC samples during compounding, at both solubilization and post-dilution of bulk solution. The in-process bulk solution is diluted, e.g., ten-fold, using a diluent of acetonitrile (ACN) and water. In some embodiments, the carfilzomib sample is diluted at least two-fold using a diluent, and in some cases, diluted eight to fifteen-fold, or eight to twelve-fold, or nine to eleven-fold, using a diluent. As used herein, an "analytical sample" is a sample that has been diluted using a diluent and can be analyzed using HPLC. In context, reference to a sample's concentration refers to the concentration of carfilzomib in the sample as assessed using an analytical sample prepared from that sample and a diluent.

Prior methods for measuring carfilzomib concentration used a diluent of 50/50 volume-to-volume (v/v) ratio of water and ACN. It has been determined herein that an excess of ACN in the diluent (> 50% volume) may lead to compromised sample solubility, which could potentially impact the IPC assay result. Moreover, as described in detail herein, it is contemplated that conventional diluents of 50/50 (v/v) ratio of water and ACN are sensitive to slight perturbations, which can impact the amount of carfilzomib in each phase, and thus can impact the accuracy of carfilzomib concentration measurements.

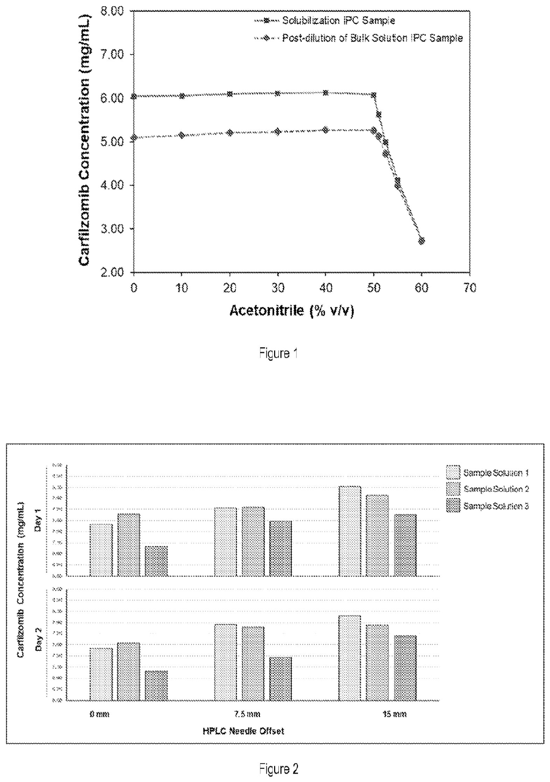

To assess the sensitivity of carfilzomib concentration measurements to the amount of ACN in the diluent, analytical samples of solubilization and post-dilution of bulk solution samples from in-process bulk (lyophilization) solution of Carfilzomib for Injection were prepared in a series of diluents ranging from 0% to 60% ACN by volume, and analyzed by HPLC using the assay as described in the examples section. In some cases, the sample is from a bulk solution from in-process bulk (lyophilization) solution of Carfilzomib for Injection. In some cases, the sample is a solubilization sample. In some cases, the sample is a post-dilution sample. In some cases, the analytical sample is refrigerated during or prior to HPLC analysis(e.g., having a temperature of 2 to 8°C). The results of the different ACN concentration conditions for carfilzomib samples are shown in Figure 1.

It is contemplated that samples may be collected at various stages of the manufacturing and formulation of carfilzomib (e.g., during carfilzomib compounding), and that methods described herein may be used to measure carfilzomib concentrations in these samples from various stages. For example, manufacturing may comprise solubilizing carfilzomib in formulated bulk solution, and may subsequently comprise diluting the formulated bulk solution (for example, by adding water for injection) to achieve a drug product comprising a target concentration of carfilzomib for filling into vials. Measurements of carfilzomib concentration may be made in the formulated bulk solution (e.g., to determine how much diluent is needed to achieve the target concentration in drug product), and again in the post-dilution of formulated bulk product (e.g., to verify that the drug product contains the target concentration or carfilzomib). As used herein, the term "solubilization sample" refers to a sample comprising solubilized carfilzomib that has not yet been diluted to a target concentration for drug product.

As used herein, the term "post-dilution of bulk solution sample" refers to a sample comprising carfilzomib bulk solution and diluent. The carfilzomib may be at, or may be a candidate for being at, a target concentration. Post-dilution bulk solution can include drug product which is used to fill vials, e.g., single dose vials.

As can be seen in Figure 1, the measured carfilzomib concentration decreases when the percentage of ACN in the diluent exceeds 50%. These data highlight a risk that excess ACN in the diluent could result in an inaccurate, low IPC assay result Thus, the methods disclosed herein include diluting the carfilzomib sample with a diluent that comprises water and less than 50% by volume ACN to form an analytical sample. In some embodiments, the diluent comprises 0.1% to 49.9% by volume ACN. In some embodiments the diluent comprises 0% to 45% by volume ACN. In some embodiments, the diluent is 25 to 40% by volume ACN. In some cases, the diluent is 30% by volume ACN. In some cases, the diluent is 40% by volume ACN. In some cases, the diluent is less than 50%, 49%, 45%, 40%, or 25% by volume ACN. In some cases, the diluent comprises ACN and is less than 50%, 49%, 45%, 40%, or 25% by volume ACN.

A series of experiments were performed to identify an alternative diluent for the IPC method for bulk (lyophilization) solution of Carfilzomib for Injection, in order to reduce the risk of an inaccurate, low IPC assay result. The data presented in Figure 1 indicate that diluents with lower % ACN pose a lower risk of an inaccurate, low IPC assay result. Two alternative diluents, 30% ACN and 40% ACN by volume, were evaluated. Each diluent was analysed across three different representative IPC assay sample concentrations, ranging from approximately 3.7 mg/mL to 7.8 mg/mL, corresponding to a range of 60% to 130% of the method nominal concentration. The resulting data is summarized in Table 1. In some cases, the carfilzomib concentration of the sample is 3 mg/mL to 8 mg/mL. In some cases, the carfilzomib concentration is 5 mg/mL to 6 mg/mL.

**Table 1**

| Acetonitrile (% v/v) | Sample Concentration Level¹ | Average Carfilzomib Concentration (mg/mL) | % RSD |
|---|---|---|---|
| 30% | | 3.68 | 0.1 |
| | 60% | 3.70 | 0.1 |
| | | 3.69 | 0.1 |
| | 100% | 6.02 | 0.2 |
| | | 6.00 | 0.1 |
| | | 6.02 | 0.2 |
| | | 7.75 | 0.1 |
| | 130% | 7.76 | 0.1 |
| | | 7.77 | 0.1 |
| | | 3.70 | 0.1 |
| | 60% | 3.69 | 0.2 |
| | | 3.72 | 1.6 |
| | | 6.04 | 0.1 |
| 40% | 100% | 6.06 | 0.1 |
| | | 6.05 | 0.1 |
| | | 7.80 | 0.4 |
| | 130% | 7.80 | 0.1 |
| | | 7.78 | 0.1 |

| | | | |
|---|---|---|---|
| ¹Relative to the method nominal concentration | | | |

For each condition evaluated, all analytical sample solutions were prepared using diluents having 30% or 40% ACN by volume, otherwise analyzed per the analytical method disclosed in the examples section below. The results demonstrated good precision as evidenced by low %RSD values. In some embodiments, the methods disclosed herein provide an RSD% of less than 2%. In various cases, the RSD% Is less than 1%. In various cases, the RSD% is less than 0.5%.

A confirmatory study was subsequently performed whereby two solubilization and two post-dilution of bulk solution IPC samples were analysed using a diluent of 40% ACN by volume. All analytical samples analysed demonstrated good precision as evidenced by the low %RSD values, and no adverse trends were observed in the results. These data are summarized in Table 2.

**Table 2**

| Sample | Average Carfilzomib Concentration (mg/mL) | % RSD |
|---|---|---|
| | 6.11 | 0.1 |
| Solubilization Sample 1 | 6.09 | 0.3 |
| | 6.09 | 0.1 |
| | 5.94 | 0.1 |
| Solubilization Sample 2 | 5.94 | 0.0 |
| | 5.95 | 0.2 |
| Post-Dilution of Bulk Solution Sample 1 | 5.20 | 0.3 |
| | 5.19 | 0.3 |
| | 5.19 | 0.4 |
| Post-Dilution of Bulk Solution Sample 2 | 4.97 | 0.2 |
| | 4.99 | 0.4 |
| | 5.00 | 0.2 |

A series of comparative analyses were performed whereby the same sample was analysed by the IPC method, using each of diluents having 50% and 40% ACN by volume, respectively for two different lots, A and B. Analytical samples from each of solubilization samples and post-dilution of bulk solution samples were analysed. Carfilzomib concentrations determined by the IPC method were comparable across both diluents. These data are shown in Table 3.

**Table 3**

| Lot | Sample | Carfilzomib Concentration (mg/mL) | |
|---|---|---|---|
| A | | 50% ACN | 40% ACN |
| | Solubilization Post-Dilution of Bulk Solution | 6.01 | 5.99 |
| | | 5.27 | 5.20 |
| B | Solubilization Post-Dilution of Bulk Solution | 6.08 | 6.05 |
| | | 5.01 | 5.03 |

The carfilzomib concentration in a solubilization or post-dilution of bulk solution sample can be used to appropriately portion out the desired amount of carfilzomib for a resulting formulation, e.g., a lyophilizate in single-dose vial. In some cases, the lyophilizate in the single-dose vial contains 10 mg carfilzomib. In some cases, the lyophilizate in the single-dose vial contains 30 mg carfilzomib. In some cases, the lyophilizate in the single-dose vial contains 60 mg carfilzomib.

### EXAMPLES

*Carfilzomib concentration assay*: An analytical sample comprising carfilzomib is assessed using a high-performance liquid chromatography (HPLC) assay. Analytical samples are prepared in triplicate. 5.0 mL of sample solution is transferred to a 50 mL volumetric flask, and diluted in sample diluent comprising the applicable ratio (v/v) of ACN and water, and mixed thoroughly. The samples are run on a Phenomenex Gemini^{™} C18 column (50 x 4.6 mm 3 µm particle size) at a column temperature of 30 °C ± 2 °C, an autosampler temperature of 5 °C ± 3 °C, a detection wavelength of 220 nm, a flow rate of 1.5 mL/min, an injection volume of 10 µL, and a total run time of 4 minutes.

Two different IPC samples of carfilzomib were assessed for concentration using varying amounts of water and ACN as dilutent, and the results are shown in the Table 4 below. The measured carfilzomib concentration was observed to drop significantly when the amount of ACN in the diluent exceeded 50%

**Table 4**

| **Diluent Composition** | **Sample 1** | **Sample 2** |
|---|---|---|
| 40% ACN | 6.00 | 5.04 |
| 50% ACN | 6.03 | 5.04 |
| 51% ACN | 5.72 | 5.01 |
| 52.5% ACN | 5.10 | 4.94 |
| 55% ACN | 4.29 | 4.14 |
| 60% ACN | 2.75 | 2.82 |

Yet another set of carfilzomib samples were assessed for concentration with diluents at varying amounts of ACN in water, and the concentration measured dropped at and above 50% ACN, as shown in Table 5.

**Table 5**

| **Diluent Composition** | **Sample 3** | **Sample 4** |
|---|---|---|
| 0% ACN | 6.04 | 5.10 |
| 10% ACN | 6.06 | 5.15 |
| 20% ACN | 6.10 | 5.21 |
| 30% ACN | 6.11 | 5.23 |
| 40% ACN | 6.13 | 5.26 |
| 50% ACN | 6.08 | 5.26 |
| 51% ACN | 5,63 | 5.13 |
| 52.5% ACN | 4.98 | 4.72 |
| 55% ACN | 4.12 | 3.98 |
| 60% ACN | 2.74 | 2.72 |

| | | |
|---|---|---|
| *Sample solutions with 50% v*/*v ACN diluent when the HPLC sample tray is cooled to* 5°C | | |

An assessment was performed on whether the temperature of the analytical sample prior to analysis by HPLC impacted the concentration of carfilzomib measured. Evidence of phase separation was observed for sample solutions prepared with 50% v/v ACN diluent at a carfilzomib concentration of about 7.8 mg/mL (Figure 2). In this case, the lowest carfilzomib concentration was detected at 0 mm needle offset (i.e., the bottom of the HPLC vial), and the highest carfilzomib concentration was detected at 15 mm needle offset (i.e., the top of the HPLC vial). This carfilzomib concentration gradient remained present to a comparable degree when the vials were re-injected the day after aliquoting (Figure 2). Importantly, this concentration gradient was not observed in any of the sample solutions prepared with 30% v/v or 40% v/v ACN diluent, nor was it detected for any sample solutions analyzed with the HPLC sample tray held at room temperature.

It is hypothesized that this differential is caused by a phase split that occurs between the ACN and water components of the diluent, and the differing solubilities of carfilzomib in these components. Due to its poor aqueous solubility, the higher carfilzomib concentration would be detected in the upper (less dense) ACN layer. Without being limited by theory, it is contemplated that sugar in the carfilzomib drug product (such as cyclodextrin, which may be used in some formulations) contributes to "sugaring out", a phenomenon in which sugar above a certain concentration threshold causes an increased concentration of ACN in the upper organic phase.

All analytical sample solutions prepared with 30% v/v ACN and 40% v/v ACN diluents yielded results with good precision as evidenced by low %RSD values when analyzed with the HPLC sample tray at 5°C. However, considerable variability was observed for analytical sample solutions prepared with 50% v/v ACN diluent, particularly with samples at 60% and 7.8 mg/mL carfilzomib concentrations (130% relative to the method nominal concentration), with the majority of these conditions having % RSD > 2.0% (Table 6).

**Table 6**

| Sample^{b} | Average Carfilzomib Concentration (mg/mL)^{a} | % RSD |
|---|---|---|
| 60% Concentration | 3.83 | *3.0* * |
| | 3.74 | *2.7* * |
| | 3.71 | 0.2 |
| 100% Concentration | 6.09 | 1.1 |
| | 6.05 | 0.0 |
| | 6.06 | 0.5 |
| 130% Concentration | 7.76 | 4.4 * |
| | 7.77 | 2.2 * |
| | 7.37 | 4.1 * |

| | | |
|---|---|---|
| *^{a} Average and % RSD calculations performed include results generated from replicates injections from the same* *HPLC vial with different HPLC needle positions (0 mm, 7.5 mm, 15 mm needle offset)* *^{b} Relative to the method nominal concentration* ** % RSD >2.0%* *Sample solutions with 40% v*/*v ACN diluent when the HPLC sample tray is cooled to 5°C* | | |

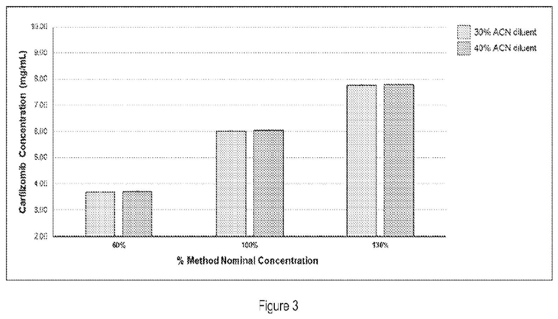

Analytical sample solutions prepared with 50% v/v ACN diluent when analyzed with the HPLC sample tray at 5°C revealed significant gradiation in the results, while all analytical sample solutions prepared with 30% and 40% v/v ACN diluents exhibited ≤2.0% RSD when analyzed under the same conditions. This observation was also true for HPLC sample vials re-injected the day after preparation. This data suggests that decreasing the percentage of ACN in the diluent by at least 10% leads to a more accurate assay result across the 70% to 130% concentration range (relative to the method nominal concentration), and that this result remains accurate even with prolonged vial storage over a 24 hour period on a cooled HPLC sample tray. Average carfilzomib concentration results and the respective % RSD values across each sample concentration prepared with 30% and 40% v/v ACN are presented in Figure 3.

The concentration of carfilzomib detected decreases when the percentage of ACN in the diluent exceeds 50%, but consistent carfilzomib concentrations (comparable to the value obtained with 50% v/v ACN diluent) were achieved with % ACN levels ranging from 50% to 0%. Sample solutions prepared using 40% v/v ACN diluent and the HPLC sample tray cooled to 5°C, revealed that the assay result remained precise and accurate across all four Kyprolis^{®} IPC samples tested. All conditions achieved %RSD ≤2.0% (Table 7).

**Table 7**

| Sample | Flask | Concentration (mg/mL)*^{a}* | % RSD a | Average Concentration (mg/mL) ^{b} | % RSD ^{b} |
|---|---|---|---|---|---|
| Solubilization Sample 1 | 1 | 6.11 | 0.1 | 6.10 | 0.2 |
| | 2 | 6.09 | 0.3 | | |
| | 3 | 6.09 | 0.1 | | |
| Post Dilution Sample 1 | 1 | 5.20 | 0.3 | 5.19 | 0.3 |
| | 2 | 5.19 | 0.3 | | |
| | 3 | 5.19 | 0.4 | | |
| Solubilization Sample 2 | 1 | 5.94 | 0.1 | 5.94 | 0.1 |
| | 2 | 5.94 | 0.0 | | |
| | 3 | 5.95 | 0.2 | | |
| Post Dilution Sample 2 | 1 | 4.97 | 0.2 | 4.99 | 0.3 |
| | 2 | 4.99 | 0.4 | | |
| | 3 | 5.00 | 0.2 | | |

| | | | | | |
|---|---|---|---|---|---|
| *^{a} Average and % RSD calculations performed include results generated from replicates injections from the same* *HPLC vial with different HPLC needle positions (0 mm, 7.5 mm, 15 mm needle offset)* *^{b} Average and % RSD calculations performed include results generated from all replicate injections (0 mm, 7.5* *mm, 15mm offset) and replicate sample preparations (Flask 1, 2, 3)* *Sample solutions with 30%*/*40%*/*50% v*/*v ACN diluent when the HPLC sample tray is at room temperature* All conditions passed % RSD sample acceptance criteria when the HPLC sample tray was held at room temperature on day 1. | | | | | |

Aspects and features of the present disclosure are set out in the following numbered clauses which contain the subject matter of the claims of the parent application as filed.
1. A method for measuring a carfilzomib concentration of a sample comprising carfilzomib comprising the steps of
   diluting the sample with a diluent comprising water and less than 50% by volume acetonitrile to form an analytical sample; and
   subjecting the analytical sample to high performance liquid chromatography (HPLC) to determine the carfilzomib concentration in the sample.
2. The method of claim 1, wherein the sample is obtained from carfilzomib compounding.
3. The method of claim 1, wherein the sample is obtained from a bulk lyophilization solution of carfilzomib.
4. The method of claim 1, wherein the sample is a solubilization sample.
5. The method of claim 1, wherein the sample is a post-dilution sample.
6. The method of any one of claims 1 to 5, wherein the diluent comprises water and 0% to 45% by volume acetonitrile.
7. The method of claim 6, wherein the diluent comprises water and 25% to 40% by volume acetonitrile.
8. The method of claim 7, wherein the diluent comprises water and 30% by volume acetonitrile.
9. The method of claim 7, wherein the diluent comprises water and 40% by volume acetonitrile.
10. The method of any one of claims 1 to 9, wherein the method comprises a relative standard deviation (RSD) value of less than 2%.
11. The method of claim 10, wherein the RSD is less than 1%.
12. The method of claim 10, wherein the RSD is less than 0.5%.
13. The method of any one of claims 1 to 12, wherein the carfilzomib concentration is 3 mg/mL to 8 mg/mL.
14. The method of claim 13, wherein the carfilzomib concentration is 5 mg/mL to 6 mg/mL.
15. The method of any one of claims 1 to 14, wherein the temperature of the analytical sample is 2°C to 8 C prior to HPLC analysis.
16. The method of any one of claims 1 to 14, wherein the temperature of the analytical sample is at room temperature prior to HPLC analysis.
17. The method of any one of claims 3 to 16, further comprising the step of preparing a lyophilizate from the sample.
18. The method of claim 17, wherein the lyophilizate contains 10 mg carfilzomib.
19. The method of claim 17, wherein the lyophilizate contains 30 mg carfilzomib.
20. The method of claim 17, wherein the lyophilizate contains 60 mg carfilzomib.

## Claims

1. A method for measuring a carfilzomib concentration of a sample comprising carfilzomib comprising the steps of
diluting the sample with a diluent comprising water and 0.1% to 49.9% by volume acetonitrile to form an analytical sample; and
subjecting the analytical sample to high performance liquid chromatography (HPLC) to determine the carfilzomib concentration in the sample.

2. The method of claim 1, wherein the sample is obtained from carfilzomib compounding.

3. The method of claim 1, wherein the sample is obtained from a bulk lyophilization solution of carfilzomib.

4. The method of claim 1, wherein the sample is a solubilization sample.

5. The method of claim 1, wherein the sample is a post-dilution sample.

6. The method of claim 1, wherein the sample comprises drug product.

7. The method of any one of claims 1 to 6, wherein the diluent comprises water and less than 49% by volume acetonitrile.

8. The method of any one of claims 1 to 7, wherein the carfilzomib concentration is 3 mg/mL to 8 mg/mL.

9. The method of claim 8, wherein the carfilzomib concentration is 5 mg/mL to 6 mg/mL.

10. The method of any one of claims 1 to 9, wherein the temperature of the analytical sample is 2°C to 8°C prior to HPLC analysis.

11. The method of any one of claims 1 to 9, wherein the temperature of the analytical sample is at room temperature prior to HPLC analysis.

12. The method of any one of claims 3 to 11, further comprising the step of preparing a lyophilizate from the sample.

13. The method of claim 12, wherein the lyophilizate contains 10 mg carfilzomib.

14. The method of claim 12, wherein the lyophilizate contains 30 mg carfilzomib.

15. The method of claim 12, wherein the lyophilizate contains 60 mg carfilzomib.
